# FASCICULE DE BREVET EUROPEEN

(11) **EP 4 581 122 B1**
(45) Date de publication et mention de la délivrance du brevet: **30.09.2026**
(21) Numéro de dépôt: 23761964.8
(22) Date de dépôt: 01.08.2023
(51) Int. Cl.: C12M 1/107, C12M 1/16, C12M 1/26

(54) **INSTALLATION DE TRAITEMENT, NOTAMMENT POUR LE TRAITEMENT PAR FERMENTATION DE DÉCHETS ORGANIQUES**
AUFBEREITUNGSANLAGE, INSBESONDERE ZUR FERMENTATIVEN AUFBEREITUNG VON ORGANISCHEN ABFÄLLEN
TREATMENT FACILITY, IN PARTICULAR FOR THE TREATMENT OF ORGANIC WASTE BY FERMENTATION

(30) Priorité: 02.09.2022 FR 2208824
(43) Date de publication de la demande: 09.07.2025
(73) Titulaire: Institut National de la Recherche pour l'Agriculture, l'Alimentation et l'Environnement, 75007 Paris (FR)
(72) Inventeur: BLONDEL, Laurent, 35530 Servon sur Vilaine (FR); DEGUEURCE, Axelle, 35630 La Chapelle-Chaussée (FR); PEU, Pascal, 35200 Rennes (FR); TREMIER, Anne, 35140 Gosné (FR)
(74) Mandataire: Ipsilon
(86) Numéro de dépôt international: PCT/FR2023/051228
(87) Numéro de publication internationale: WO 2024/047302

(56) Documents cités:
- CN-A- 102 816 690
- FR-A1- 3 097 230
- US-A1- 2009 305 377

## Description

La présente invention concerne une installation de traitement, notamment pour le traitement par fermentation, en particulier anaérobie, de produits solides au moins partiellement fermentescibles, en particulier de déchets organiques solides, en vue notamment de la production de biogaz.

On entend par biogaz, un gaz combustible produit par la fermentation de matières organiques en l'absence d'oxygène.

Elle concerne plus particulièrement une installation de traitement comprenant un réacteur tubulaire, un châssis support dudit réacteur tubulaire, et au moins une cassette présentant une zone ajourée de stockage de produit, ledit réacteur tubulaire étant équipé au moins d'une entrée d'alimentation en produits à traiter, d'une première sortie , dite sortie d'évacuation des produits traités, d'une deuxième sortie , dite sortie d'évacuation de biogaz, d'un orifice d'entrée de liquide, dit orifice d'entrée d'un inoculum, d'un orifice de sortie de liquide, dit orifice de sortie de lixiviat, ledit réacteur tubulaire comprenant un corps tubulaire et un séparateur liquide/solide, ledit corps tubulaire étant fermé à chacune de ses extrémités par un dispositif de fermeture étanche à l'air, ce dispositif de fermeture comprenant au moins un élément de fermeture monté mobile entre une position ouverte et une position fermée de ladite extrémité, lesdites extrémités du corps formant l'une, l'entrée d'alimentation en produits à traiter, l'autre, la sortie d'évacuation des produits traités dudit réacteur, et la ou chaque cassette étant montée déplaçable axialement à coulissement à l'intérieur du corps depuis l'entrée d'alimentation en produits à traiter vers la sortie d'évacuation des produits traités.

La transformation de matières organiques en biogaz, tel que du méthane, par fermentation anaérobie desdites matières organiques, est un procédé biologique bien connu à ceux versés dans cet art. Cette fermentation anaérobie, parfois encore appelée méthanisation, est une technologie éprouvée pour la valorisation des effluents organiques liquides, mais reste encore perfectible pour la valorisation des déchets organiques solides. L'un des problèmes, notamment, du traitement de déchets organiques solides réside dans la difficulté d'une mise en contact des microorganismes et des nutriments dans un environnement où l'agitation mécanique est difficile. Les installations de méthanisation basées sur le recyclage de déchets organiques solides se développent néanmoins de plus en plus. En particulier, un véritable besoin existe concernant les installations de méthanisation dites de proximité. Ces installations de traitement des déchets organiques solides par fermentation anaérobie, sont dites de proximité, par opposition aux installations de traitement industrielles. Ces installations de traitement de proximité sont destinées, par opposition aux installations industrielles, au traitement annuel d'une quantité plus faible de déchets, généralement de l'ordre de quelques centaines de tonnes de déchets par an. Il en résulte des contraintes différentes de ces installations de traitement de proximité à savoir notamment : un faible encombrement, une maintenance réduite, une utilisation simplifiée, une production élevée de biogaz associée à une faible consommation d'eau et d'énergie. Jusqu'à présent, les installations de traitement des déchets organiques solides par fermentation anaérobie disponibles sur le marché, telles que celle décrite dans le brevet FR 3 097 230, ne donnent pas entière satisfaction au regard des exigences mentionnées ci-dessus.

Un but de l'invention est de proposer une installation de traitement du type précité dont la conception permet une production élevée de biogaz associée à la production d'une quantité réduite de digestat sans nuire à la facilité d'utilisation de l'installation et à une faible consommation en eau.

Un autre but de l'invention est de proposer une installation de traitement du type précité dont la conception permet un accès facilité au séparateur liquide/solide tout en améliorant les performances dudit séparateur.

A cet effet, l'invention a pour objet une installation de traitement, notamment pour le traitement par fermentation de produits solides au moins partiellement fermentescibles, en particulier de déchets organiques solides, en vue notamment de la production de biogaz, ladite installation comprenant un réacteur tubulaire, un châssis support dudit réacteur tubulaire, et au moins une cassette présentant une zone ajourée de stockage de produit, ledit réacteur tubulaire étant équipé au moins d'une entrée d'alimentation en produits à traiter, d'une première sortie, dite sortie d'évacuation des produits traités, d'une deuxième sortie, dite sortie d'évacuation de biogaz, d'un orifice d'entrée de liquide, dit orifice d'entrée d'un inoculum, d'un orifice de sortie de liquide, dit orifice de sortie de lixiviat, ledit réacteur tubulaire comprenant un corps tubulaire et un séparateur liquide/solide, ledit corps tubulaire étant fermé à chacune de ses extrémités par un dispositif de fermeture étanche à l'air, ce dispositif de fermeture comprenant au moins un élément de fermeture monté mobile entre une position ouverte et une position fermée de ladite extrémité, lesdites extrémités du corps formant l'une, l'entrée d'alimentation en produits à traiter, l'autre, la sortie d'évacuation des produits traités dudit réacteur, et la ou chaque cassette étant montée déplaçable axialement à coulissement à l'intérieur du corps depuis l'entrée d'alimentation en produits à traiter vers la sortie d'évacuation des produits traités, caractérisée en ce que le séparateur liquide/solide comprend un filtre présentant une partie active de filtration configurée pour, en position fermée de l'élément mobile de fermeture de la sortie d'évacuation des produits traités dudit réacteur et à l'état positionné d'au moins une cassette dans le réacteur, s'interposer entre les orifices d'entrée d'un inoculum et de sortie de lixiviat et la zone de stockage de la ou de chaque cassette disposée à l'intérieur du réacteur, et en ce que l'orifice d'entrée d'un inoculum, l'orifice de sortie de lixiviat et au moins une partie du filtre sont portés par l'élément mobile de fermeture de la sortie d'évacuation des produits traités dudit réacteur.

La partie active de filtration du filtre est donc configurée pour, en position fermée de l'élément mobile de fermeture de la sortie d'évacuation des produits traités dudit réacteur et à l'état positionné d'au moins une cassette dans le réacteur, s'interposer, c'est-à-dire former une barrière filtrante, entre les orifices d'entrée d'un inoculum et de sortie de lixiviat et la zone de stockage de la ou de chaque cassette disposée à l'intérieur du réacteur. Ainsi, tout inoculum amené dans le réacteur doit franchir la barrière filtrante avant de parvenir à la zone de stockage de produits à traiter de chaque cassette, de même que tout lixiviat issu de la zone de stockage de produits de la ou des cassettes disposées à l'intérieur du réacteur doit franchir la barrière filtrante avant de pouvoir être évacué du réacteur. Comme cette barrière filtrante est active pour la filtration de l'inoculum et du lixiviat qui circulent suivant deux sens opposés, cette barrière filtrante peut s'autonettoyer sous l'action de cette circulation à double sens. Ainsi, la formation d'un gâteau de filtration est ralentie. En d'autres termes, le filtre du séparateur liquide/solide du réacteur présente une partie active de filtration avec une première surface configurée pour, en position fermée des éléments de fermeture et à l'état inséré d'au moins une cassette dans le réacteur, être tournée vers la zone de stockage de produit de la ou de chaque cassette positionnée à l'intérieur du réacteur et une seconde surface opposée, et l'orifice d'entrée d'un inoculum et l'orifice de sortie de lixiviat sont disposés côté seconde surface dudit filtre. La conception du séparateur liquide/solide permet d'assurer, à chaque opération d'immersion, un décolmatage du filtre lors de l'amenée de l'inoculum. L'intégration d'au moins une partie du filtre du séparateur liquide/solide dans l'élément mobile de fermeture de la sortie d'évacuation des produits traités du réacteur permet un accès facilité au filtre pour son nettoyage ou son remplacement. La conception de l'installation permet un fonctionnement de l'installation avec une intervention manuelle réduite.

Selon un mode de réalisation de l'invention, l'orifice d'entrée d'un inoculum, l'orifice de sortie de lixiviat sont communs et formés par un même orifice.

Il en résulte une simplification de l'installation sans nuire à son fonctionnement. Cette disposition permet de disposer d'un circuit d'amenée de l'inoculum au réacteur commun avec le circuit d'évacuation du lixiviat du réacteur de sorte que le circuit est soumis à deux sens de circulation, ce qui permet de limiter également l'encrassement du circuit.

Selon un mode de réalisation de l'invention, le corps tubulaire du réacteur est un corps cylindrique de révolution et, en position fermée de l'élément mobile de fermeture de la sortie d'évacuation des produits traités du réacteur, l'axe longitudinal central du corps tubulaire cylindrique du réacteur passe par l'orifice d'entrée d'un inoculum et l'orifice de sortie de lixiviat. En d'autres termes, en position fermée de l'élément mobile de fermeture de la sortie d'évacuation des produits traités du réacteur, l'orifice d'entrée, l'orifice de sortie et le corps du réacteur sont coaxiaux.

Selon un mode de réalisation de l'invention, l'installation comprend un réservoir de stockage de liquide disposé à l'extérieur du réacteur et un circuit de circulation de fluide pour le raccordement dudit réservoir à l'orifice d'entrée d'un inoculum et à l'orifice de sortie de lixiviat. A nouveau, lorsque les orifices sont communs, il en résulte une simplification de l'installation et un ralentissement de la vitesse d'encrassement du circuit.

Selon un mode de réalisation de l'invention, l'installation comprend une pompe à inversion de sens disposée sur ledit circuit de circulation de fluide et une unité de pilotage de ladite pompe. Le traitement peut ainsi s'opérer par simple remplissage en inoculum du réacteur jusqu'à immersion totale des cassettes contenues dans le réacteur, maintien de l'immersion pendant une durée prédéterminée puis vidange du réacteur.

Selon un mode de réalisation de l'invention, à l'état fermé du réacteur et inséré de la ou les cassettes dans le réacteur, la partie du filtre portée par l'élément mobile de fermeture de la sortie d'évacuation des produits traités dudit réacteur se prolonge par un conduit axial poreux disposé à l'intérieur du réacteur. Cette conception permet d'augmenter la surface de filtration et d'atteindre plus rapidement la zone de stockage de produits à traiter de la ou chaque cassette disposée à l'intérieur du réacteur.

Selon un mode de réalisation, de l'invention, la ou au moins l'une des cassettes est une cassette cylindrique munie intérieurement d'une conduite centrale s'étendant au moins depuis une extrémité du cylindre jusqu'à l'extrémité opposée.

Selon un mode de réalisation, la conduite centrale de la cassette est une conduite poreuse, ladite conduite formant au moins une partie du conduit axial poreux disposé à l'intérieur du réacteur. Cette configuration permet de renouveler une partie du filtre à chaque évacuation d'une cassette du réacteur et remplacement de ladite cassette par une nouvelle cassette. Il en résulte à nouveau une efficacité accrue de la filtration.

Selon un mode de réalisation de l'invention, ladite cassette cylindrique comprend un corps cylindrique délimité par une paroi ajourée et au moins deux couvercles ajourés de fermeture l'un, d'une extrémité, l'autre de l'extrémité opposée du cylindre formé par le corps, la paroi de délimitation du corps est écartée de la conduite centrale pour définir un espace formant la zone ajourée de stockage de produit de la cassette, et les extrémités de la conduite centrale sont de formes complémentaires. Le fait que les extrémités de la conduite centrale sont de formes complémentaires permet une liaison par emboîtement des conduites centrales d'au moins deux cassettes cylindriques identiques ou similaires, c'est-à-dire avec les mêmes formes des extrémités de la conduite centrale. La réalisation des extrémités de la conduite centrale avec des formes complémentaires permet d'assurer de manière aisée une continuité du conduit axial d'une cassette à une autre, la conduite centrale de chaque cassette formant un tronçon du conduit axial. Cette conception permet également, lorsque le réacteur contient plusieurs cassettes, d'obtenir un alignement parfait des cassettes qui, à l'état inséré dans le corps du réacteur, sont disposées alignées l'une à la suite de l'autre à l'intérieur dudit corps de réacteur.

Selon un mode de réalisation de l'invention, le filtre présente des pores dont l'aire d'ouverture est prédéterminée et chaque ajour de la paroi de délimitation du corps de la cassette et chaque ajour des couvercles de fermeture présentent une aire d'ouverture supérieure à l'aire d'ouverture de l'un quelconque des pores du filtre.

Selon un mode de réalisation de l'invention, la partie du filtre portée par l'élément mobile de fermeture de la sortie d'évacuation des produits traités dudit réacteur est formée de deux cônes à conicité inversée pour se raccorder par la base des cônes, les sommets des cônes étant disposés sur une ligne formant l'axe longitudinal central du corps tubulaire du réacteur en position fermée de l'élément mobile de fermeture de la sortie d'évacuation des produits traités. Cette conception permet de limiter la formation d'un gâteau de filtration.

Selon un mode de réalisation de l'invention, l'un des cônes formant au moins une partie de la partie active de filtration du filtre est délimité par une surface poreuse, l'autre cône est délimité par une surface pleine et les orifices d'entrée d'un inoculum et de sortie de lixiviat sont disposés au niveau du sommet du cône à surface pleine.

Selon un mode de réalisation de l'invention, l'installation comprend au moins une pièce amovible configurée pour, à l'état positionné à l'intérieur du corps du réacteur et, en position fermée de l'élément mobile de fermeture de la sortie d'évacuation des produits traités, former une pièce entretoise entre la partie du filtre portée par l'élément mobile de fermeture de la sortie d'évacuation des produits traités dudit réacteur et la ou les cassettes disposées à l'intérieur du réacteur. La présence de cette pièce entretoise facilite le positionnement relatif de la partie active de filtration et de la cassette la plus proche de la partie active de filtration.

L'invention a encore pour objet un procédé de traitement, notamment de traitement par fermentation de produits solides au moins partiellement fermentescibles, en particulier de déchets organiques solides, en vue notamment de la production de biogaz, à l'aide d'une installation du type précité, caractérisé en ce que le procédé comprend, à l'état positionné d'une ou plusieurs cassettes à l'intérieur du réacteur et en position fermée des éléments de fermeture du réacteur, une étape d'alimentation du réacteur en inoculum à travers l'orifice d'entrée d'un inoculum dudit réacteur jusqu'à immersion de la ou des cassettes dans l'inoculum, une étape de maintien de l'inoculum à l'intérieur du réacteur pendant une durée prédéterminée et une étape de vidange du réacteur en lixiviat à travers l'orifice de sortie de lixiviat dudit réacteur.

### Brève description des dessins

L'invention sera bien comprise à la lecture de la description suivante d'exemples de réalisation, en référence aux dessins annexés dans lesquels :
[Fig. 1] représente une vue en perspective d'une installation conforme à l'invention,
[Fig. 2] représente une vue partielle en coupe d'une installation conforme à l'invention en position fermée des éléments de fermeture,
[Fig. 3] représente une vue partielle en perspective prise depuis une extrémité du réacteur,
[Fig. 4] représente une vue partielle en coupe d'une installation conforme à l'invention au cours du chargement d'une cassette en parallèle du déchargement d'une cassette,

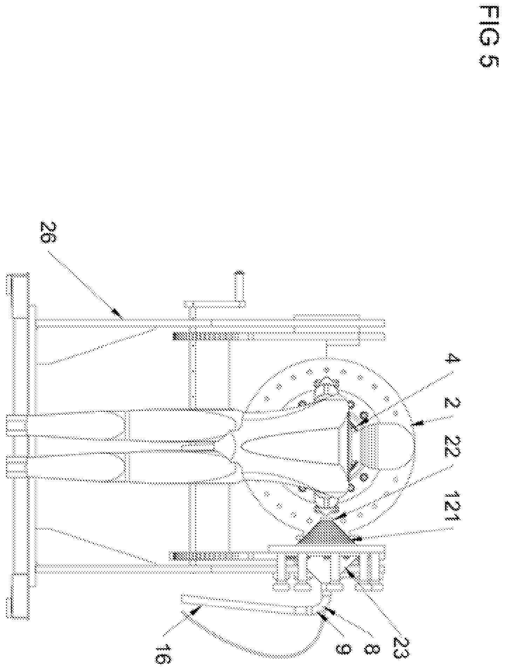

[Fig. 5] représente une vue partielle en perspective de l'installation prise côté sortie d'évacuation des produits traités du réacteur,
[Fig. 6] représente une vue partielle en coupe du réacteur d'une installation conforme à l'invention en l'absence de cassette à l'intérieur du réacteur,

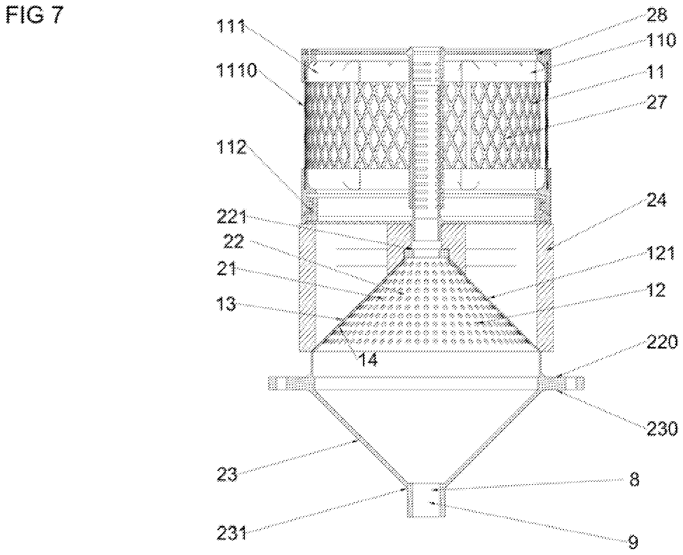

[Fig. 7] représente une vue partielle en coupe du séparateur liquide/solide associé à une pièce entretoise et à une cassette,

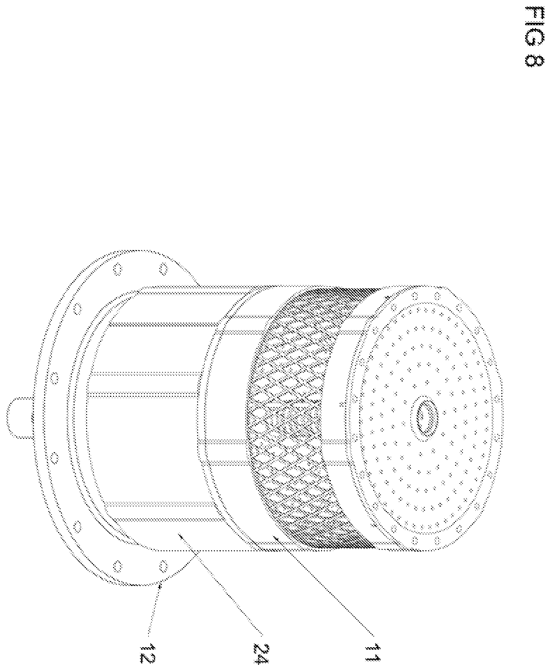

[Fig. 8] représente une vue partielle en perspective du séparateur liquide/solide associé à une pièce entretoise et à une cassette,
[Fig. 9] représente une vue en perspective d'une cassette à l'état ouvert,
[Fig. 10] représente une vue en coupe d'une cassette à l'état fermé,
[Fig. 11] représente une vue en transparence de la pièce entretoise,
[Fig. 12] représente une vue en coupe de la pièce entretoise,
[Fig. 13] représente une vue en position éclatée d'une pièce entretoise.

Comme mentionné ci-dessus, l'installation 1 de traitement objet de l'invention est plus particulièrement destinée au traitement par fermentation anaérobie de déchets organiques solides, tels que des déchets urbains, en particulier des déchets de cuisine des ménages, de restaurateurs, de marché comprenant les déchets de préparation (pelures, pulpes) jusqu'aux restes de repas, à raison, de préférence, de 50 à 200 tonnes par an.

Cette installation permet en particulier, à partir de cette fermentation de matières organiques en l'absence d'oxygène, la production de gaz combustibles composés essentiellement de méthane ou de dioxyde de carbone et appelés biogaz.

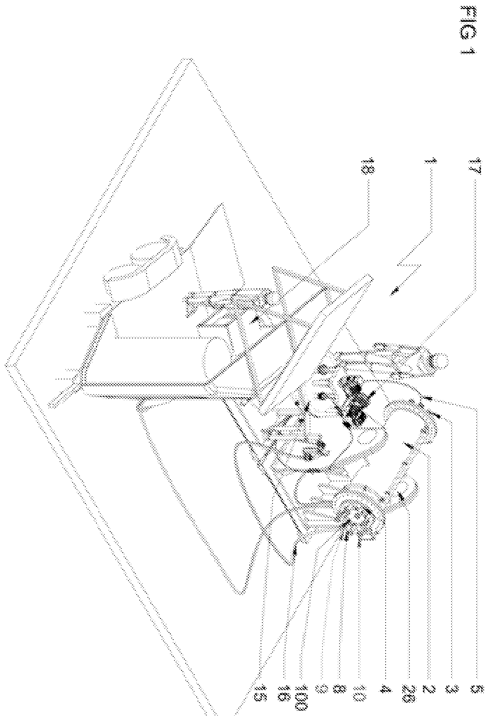

Cette installation 1, représentée à la figure 1, comprend un réacteur 2 tubulaire et un châssis support 26 dudit réacteur 2 tubulaire.

Le réacteur 2 tubulaire comprend un corps 6 tubulaire et un séparateur 7 liquide/solide. Le corps 6 tubulaire est un corps allongé qui peut être de section transversale quelconque. Idéalement, ce corps 6 tubulaire est un corps cylindrique de révolution. La section dudit corps est une section circulaire.

Ce corps 6 tubulaire est fermé à chacune de ses extrémités par un dispositif 10 de fermeture étanche à l'air. Chaque dispositif 10 de fermeture comprend un élément 100 de fermeture, tel qu'une porte ou un hublot, monté mobile entre une position ouverte et une position fermée de ladite extrémité. Cet élément 100 de fermeture est, pour son passage d'une position à une autre, articulé au corps 6 tubulaire du réacteur 2 par une liaison articulée, telle qu'une liaison charnière.

Le réacteur 2 présente une entrée 3 d'alimentation en produits à traiter, formée par l'une des extrémités du corps 6, une sortie 4 d'évacuation des produits traités formés par l'extrémité opposée du corps 6 tubulaire et une sortie 5 d'évacuation de biogaz.

Il doit être noté que l'entrée 3 d'alimentation en produits à traiter et la sortie 4 d'évacuation des produits traités peuvent être interverties selon les modalités d'utilisation.

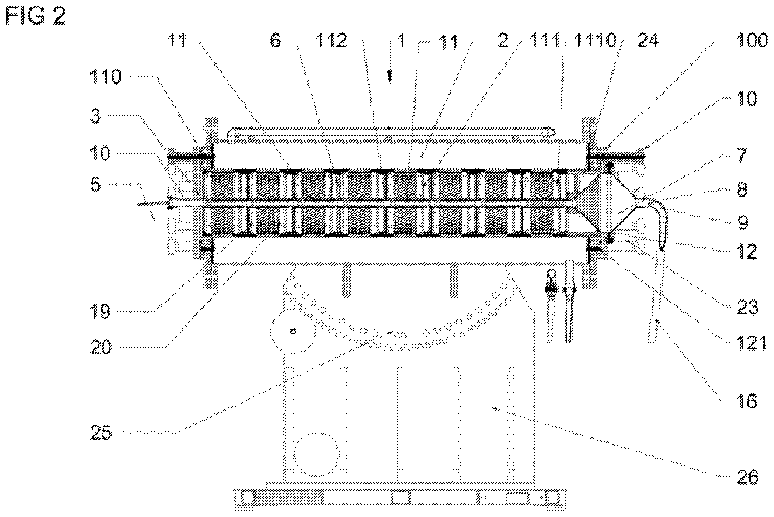

La sortie 5 d'évacuation de biogaz est, comme illustrée à la figure 2, une sortie axiale disposée au niveau de l'élément 100 de fermeture de l'entrée 3 d'alimentation en produits à traiter.

Le corps 6 du réacteur 2 tubulaire est, sur au moins une partie de sa longueur, délimité par une double paroi et est équipé de moyens de chauffage.

Ces moyens de chauffage sont, dans l'exemple représenté à la figure 1, formés par un circuit d'eau chaude dont au moins une partie est formée par l'espace laissé libre entre les deux parois de la double paroi du corps 6 du réacteur tubulaire.

Ce circuit de chauffage est alimenté en fluide chaud à partir d'un réservoir de fluide chaud disposé à proximité du châssis support 26. Ce réservoir de fluide chaud est équipé d'organes de chauffage dudit fluide, tels que des résistances électriques.

Dans les exemples représentés, le réacteur 2 est monté sur le châssis support 26 de manière réglable en inclinaison, par l'intermédiaire d'une liaison pivot entre le châssis support 26 et le réacteur 2, pour faire varier la différence de niveau entre l'entrée 3 d'alimentation en produits à traiter et la sortie 4 d'évacuation des produits traités dudit réacteur 2. Cette liaison pivot présente un axe pivot dit horizontal s'étendant transversalement à l'axe longitudinal du corps 6 du réacteur et à l'horizontale à l'état positionné de ladite installation sur une surface plane horizontale.

Cette possibilité d'inclinaison du réacteur 2 tubulaire par déplacement à pivotement autour d'un axe pivot dit horizontal présente un grand nombre d'avantages. Elle permet de réduire l'encombrement du réacteur, elle autorise un déplacement gravitaire à l'intérieur dudit réacteur et elle facilite le rechargement en produits à traiter du réacteur.

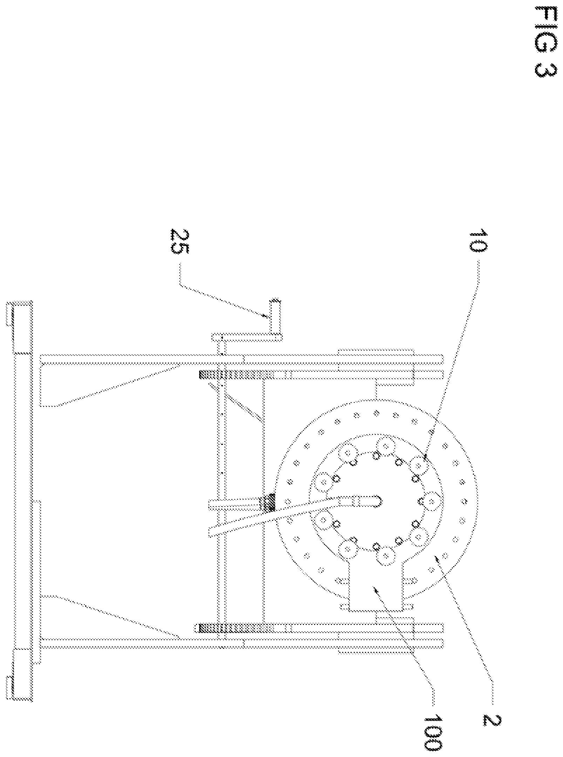

Pour faciliter ce déplacement à pivotement du réacteur 2 par rapport au châssis support 26, l'installation peut comprendre un système 25 d'entraînement en déplacement à pivotement du réacteur 2 par rapport au châssis support à actionnement manuel ou automatique. Ce système 25 d'entraînement est partiellement visible aux figures 2 et 3.

Le pivot par lequel passe l'axe pivot de la liaison pivot entre le châssis support 26 et le réacteur 2 est monté solidaire en rotation d'une roue crantée. Cette roue crantée vient en prise avec un pignon entraîné par une manivelle et porté par le châssis support 26. La roue dentée, le pignon et la manivelle forment le système 25 d'entraînement.

Pour permettre l'alimentation en produits à traiter du réacteur, l'installation 1 de traitement comprend au moins une, de préférence, plusieurs cassettes 11. Chaque cassette 11 est introduite dans le réacteur 2 par l'entrée 3 d'alimentation en produits à traiter et extraite du réacteur 2 par la sortie 4 d'évacuation de produits traités. Chaque cassette 11 est configurée, c'est-à-dire conformée et dimensionnée, pour être déplaçable axialement à coulissement à l'intérieur du corps 6 du réacteur 2 depuis l'entrée 3 d'alimentation en produits à traiter vers la sortie 4 d'évacuation des produits traités.

Les cassettes 11 sont configurées pour être disposées l'une derrière l'autre à l'état aligné à l'intérieur du corps 6 du réacteur 2. Elles forment alors une ligne de cassettes, de sorte qu'une poussée exercée sur la ligne, à une extrémité de la ligne de cassettes entraîne le déplacement des cassettes de l'ensemble de la ligne.

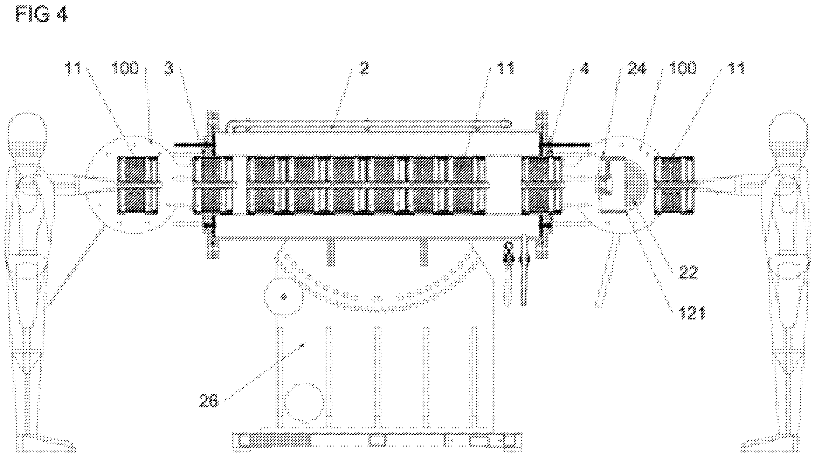

Cette disposition permet la sortie d'une cassette d'une extrémité du corps 6 du réacteur 2, en parallèle de l'entrée d'une cassette par l'extrémité opposée du corps 6 du réacteur 2, comme illustré à la figure 4.

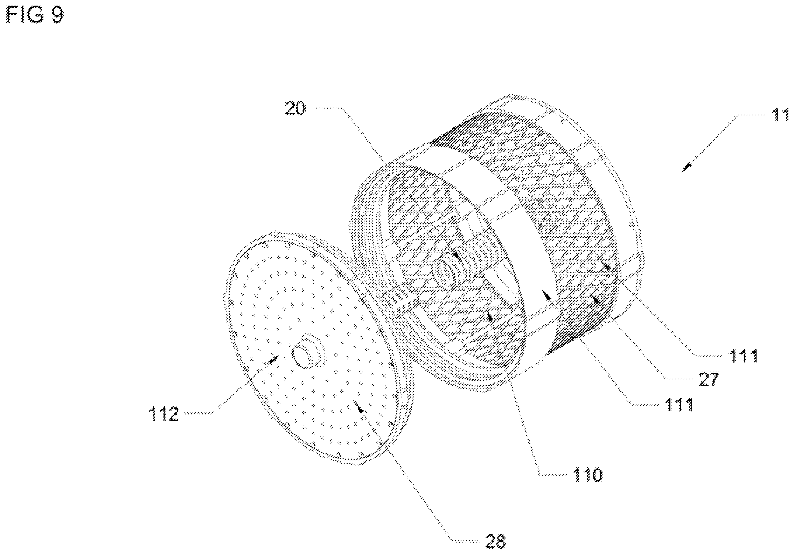

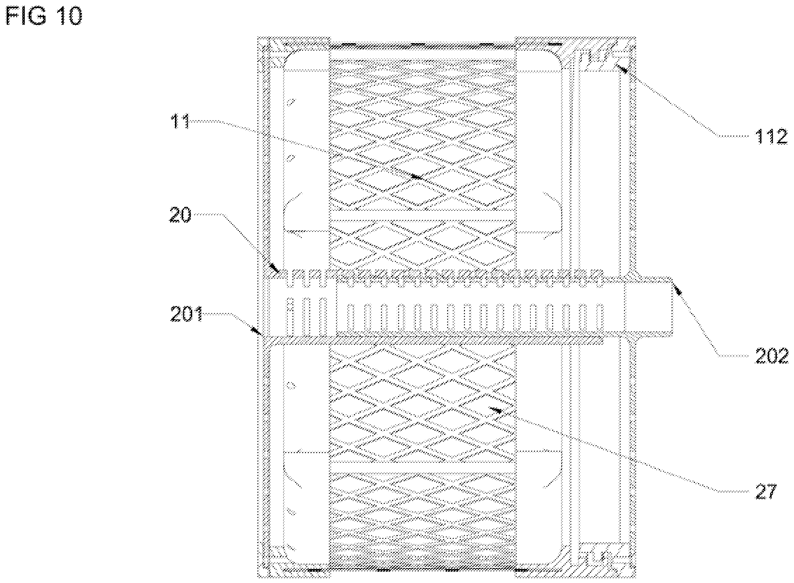

Chaque cassette 11, telle que représentée aux figures 9 et 10, est une cassette cylindrique munie intérieurement d'une conduite 20 centrale s'étendant au moins depuis une extrémité du cylindre jusqu'à l'extrémité opposée. Cette cassette 11 cylindrique comprend un corps 111 cylindrique délimité par une paroi 1110 ajourée et au moins deux couvercles 112 ajourés de fermeture, l'un, d'une extrémité, l'autre, de l'extrémité opposée du cylindre formé par le corps 111.

Une partie de la conduite 20 centrale est réalisée d'une seule pièce avec l'un des couvercles 112 ajouré et une autre partie de la conduite 20 centrale est réalisée d'une seule pièce avec l'autre couvercle 112.

Lesdites parties de conduite centrale sont reliées l'une à l'autre par emboîtement lors de la fermeture par vissage de l'un des couvercles 112 au corps de la cassette.

Ainsi, une continuité de la conduite centrale est assurée au niveau de la cassette 11. Dans les exemples représentés, la partie ajourée du corps de la cassette est formée par un grillage et chaque ajour 27 du corps correspond à une maille du grillage.

Les couvercles 112 ajourés présentent, quant à eux, des ajours 28 formés par des perforations.

On note que les extrémités de la conduite 20 centrale, représentées en 201 et 202 aux figures, sont de forme complémentaire pour permettre une liaison par emboîtement des conduites 20 centrales d'au moins deux cassettes cylindriques identiques ou similaires.

Une extrémité de la conduite 20 centrale fait saillie de l'un des couvercles 112 en direction de l'extérieur de la cassette pour faciliter cet emboîtement. A l'état positionné des cassettes 11 alignées à l'intérieur du réacteur 2, les conduites 20 centrales des cassettes forment une conduite continue coaxiale à l'axe du corps 6 du réacteur 2.

La paroi 1110 de délimitation du corps de chaque cassette 11 est écartée de la conduite 20 centrale pour définir un espace formant la zone ajourée de stockage de produits de la cassette. Cette zone ajourée de stockage est représentée en 110 aux figures.

Les produits à traiter sont donc, après ouverture de l'un des couvercles de la cassette, introduits dans le corps de la cassette dans l'espace entre la conduite 20 centrale et la paroi 1110 périphérique de délimitation du corps 111 cylindrique de la cassette 11. La cassette 11 peut, après refermeture du couvercle, être introduite dans le réacteur 2.

Si nécessaire, pour faciliter le déplacement des produits à traiter, et en particulier le déplacement de chaque cassette à l'intérieur du réacteur 2, il peut être prévu un pousseur monté mobile à coulissement à l'intérieur du réacteur 2.

Grâce à ce pousseur, chaque cassette est, à l'état positionné dans le conduit du séparateur liquide/solide, déplaçable à coulissement dans ledit conduit du séparateur liquide/solide, sous l'action d'une poussée exercée par ledit pousseur sur ladite cassette.

Ce pousseur est, lorsqu'il est présent, un pousseur extractible, qui peut être extrait ou introduit dans le réacteur 2 par l'entrée 3 d'alimentation en produits à traiter du réacteur 2.

Le réacteur 2 comprend encore un séparateur 7 liquide/solide et est équipé d'un orifice 8 d'entrée de liquide, appelé orifice d'entrée d'un inoculum, et d'un orifice 9 de sortie de liquide, appelé orifice 9 de sortie de lixiviat.

Le séparateur 7 liquide/solide comprend un filtre 12 qui présente une partie active de filtration 121 configurée pour, en position fermée de l'élément 100 mobile de fermeture de la sortie 4 d'évacuation des produits traités du réacteur 2, et à l'état positionné d'au moins une cassette 11 dans le réacteur 2, s'interposer entre les orifices 8 d'entrée d'un inoculum et 9 de sortie de lixiviat, et la zone 110 de stockage de la ou des cassettes 11 disposées à l'intérieur du réacteur.

Cette partie active de filtration 121 forme donc une barrière filtrante avec d'un côté de ladite barrière les orifices 8 d'entrée d'un inoculum et 9 de sortie de lixiviat, et de l'autre côté de la barrière filtrante, la zone 110 de stockage de la ou chaque cassette 11 disposée à l'intérieur du réacteur 2.

De manière également caractéristique à l'invention, l'orifice 8 d'entrée d'un inoculum, l'orifice 9 de sortie du lixiviat et au moins une partie du filtre 12 sont portés par l'élément 100 mobile de fermeture de la sortie 4 d'évacuation des produits traités du réacteur 2, comme cela est visible à la figure 2.

La partie active de filtration 121 du filtre 12 présente donc une première surface 13 tournée vers la zone 110 de stockage de produits de la ou des cassettes 11, à l'état positionné de la ou des cassettes à l'intérieur du réacteur 2, et une seconde surface 14 opposée.

L'orifice 8 d'entrée d'un inoculum et l'orifice 9 de sortie de lixiviat sont disposés côte seconde surface 14 dudit filtre 12.

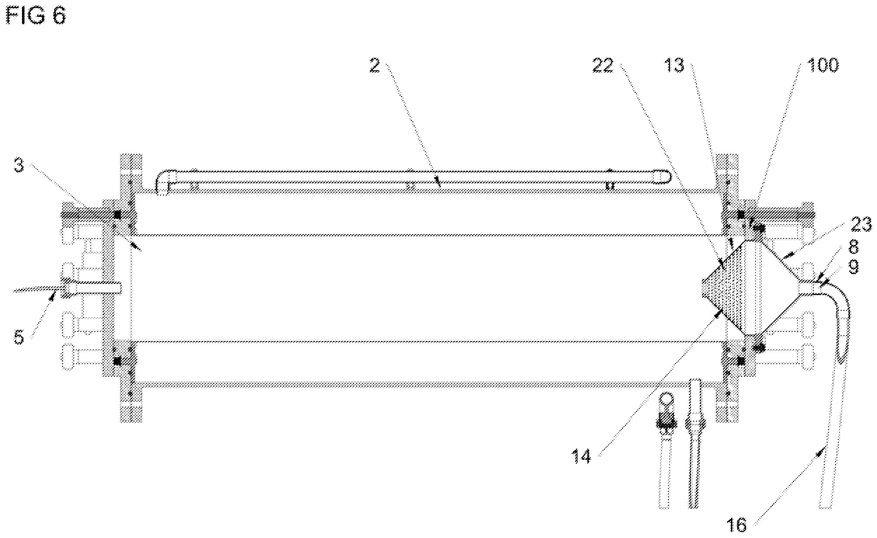

Dans l'exemple tel qu'illustré à la figure 6, la partie du filtre 12 portée par l'élément 100 mobile de fermeture de la sortie 4 d'évacuation des produits traités du réacteur 2, est formée de deux cônes 22, 23 à conicité inversée, pour se raccorder par la base 220, 230 des cônes.

Les sommets 221 et 231 des cônes 22 et 23 sont, en position fermée de l'élément mobile de fermeture de la sortie 4 d'évacuation des produits traités du réacteur, disposés sur une ligne formant l'axe longitudinal central du corps 6 tubulaire du réacteur 2.

L'un des cônes, représenté en 22 aux figures, qui forme au moins une partie de la partie active de filtration 121 du filtre 12, est délimité par une surface poreuse. L'autre cône 23 est délimité par une surface pleine.

Les orifices 8 d'entrée d'un inoculum et 9 de sortie de lixiviat sont disposés au niveau du sommet 231 du cône 23 à surface pleine. C'est cet orifice 8 d'entrée d'un inoculum et cet orifice 9 de sortie de lixiviat qui sont communs et ici formés par un même orifice.

En position fermée de l'élément 100 mobile de fermeture de la sortie 4 d'évacuation des produits traités du réacteur, l'axe longitudinal central du corps 6 tubulaire cylindrique du réacteur 2 passe par l'orifice 8 d'entrée d'un inoculum et l'orifice 9 de sortie de lixiviat.

On note que le cône 22, délimité par une surface poreuse, est, en position fermée de l'élément 100 de fermeture de la sortie 4 d'évacuation des produits traités, tourné vers l'intérieur du réacteur 2 et fait saillie à l'intérieur du corps 6 du réacteur 2. Pour permettre ce positionnement, la liaison articulée de l'élément 100 de fermeture au corps 6 du réacteur est une liaison avec au moins 2 degrés de liberté pour autoriser un déplacement à pivotement et en translation de l'élément 100 de fermeture lors de son passage d'une position à une autre. Le cône 23 délimité par une surface plane est, quant à lui, tourné vers l'extérieur du réacteur en position fermée dudit réacteur.

Pour permettre une alimentation en inoculum au niveau de l'orifice 8 d'entrée d'un inoculum du réacteur et une récupération du lixiviat évacué du réacteur par l'orifice 9 de sortie de lixiviat, l'installation comprend un réservoir 15 de stockage de liquide disposé à l'extérieur du réacteur, et un circuit 16 de circulation de fluide pour le raccordement du réservoir 15 à l'orifice 8 d'entrée d'un inoculum et à l'orifice 9 de sortie de lixiviat.

L'installation 1 comprend encore une pompe 17 à inversion de sens disposée sur ledit circuit 16 de circulation de fluide et une unité 18 de pilotage de ladite pompe.

Le réservoir 15 de stockage de liquide peut être prérempli en inoculum lors de la mise en route de l'installation. Cet inoculum peut être composé de tout digestat liquide en provenance d'une unité de méthanisation située à proximité. De préférence, le digestat utilisé comme inoculum provient d'un site traitant en majorité des biodéchets de cuisine.

Les produits à traiter contenus dans le réacteur fermentent, notamment au contact de l'inoculum, et se liquéfient au moins partiellement.

Cette fraction liquide et l'inoculum injecté forment le lixiviat. Ce lixiviat est pompé en dehors du réacteur par la pompe 17 ayant servi à l'injection de l'inoculum, et est amené au réservoir 15 de stockage de liquide avant de constituer l'inoculum qui sera injecté lors d'un nouveau cycle d'immersion.

Chaque cycle d'immersion comprend donc une étape d'alimentation en inoculum du réacteur en une quantité fonction d'un niveau de remplissage souhaité du réacteur. Généralement, cette quantité correspond à la quantité nécessaire pour que les zones de stockage des cassettes contenues dans le réacteur soient immergées dans cet inoculum.

L'étape d'alimentation est suivie d'une étape de maintien de l'inoculum dans le réacteur pendant une durée déterminée. Cette seconde étape est suivie d'une étape de vidange du réacteur pour récupérer le lixiviat dans le réservoir 15 de stockage de liquide. L'ensemble du cycle d'immersion peut s'opérer avec une seule pompe 17 et un seul réservoir 15 de stockage.

Ce réservoir 15 de stockage peut être équipé d'un système de vidange ou de trop-plein pour maîtriser le niveau de remplissage dudit réservoir. Le lixiviat soutiré de ce réservoir 15 de stockage peut être valorisé.

Pour parfaire l'installation, on note que dans l'exemple de la figure 2, c'est-à-dire à l'état fermé du réacteur 2 et inséré de la ou des cassettes 11 dans le réacteur 2, la partie du filtre 12 portée par l'élément 100 mobile de fermeture de la sortie 4 d'évacuation des produits traités du réacteur 2, se prolonge par un conduit 19 axial poreux disposé à l'intérieur du réacteur 2.

Ce conduit axial poreux est formé de tronçons de conduits avec chaque tronçon lui-même formé par la conduite 20 centrale d'une cassette 11, cette conduite 20 centrale étant poreuse.

De préférence, le filtre 12 présente des pores 21 dont l'aire d'ouverture est prédéterminée. Chaque ajour 27 de la paroi 1110 de délimitation du corps 111 de la cassette 11 et chaque ajour 28 des couvercles 112 de fermeture présentent une aire d'ouverture supérieure à l'aire d'ouverture de l'un quelconque des pores du filtre 12.

Ainsi, à titre d'exemple, chaque ajour 27 de la paroi 1110 de délimitation du corps 111 de la cassette, présente une aire au moins égale à 22,5 mm² et chaque ajour 28 des couvercles 112 de fermeture présente une aire d'ouverture au moins égale à 12,6 mm².

Les pores du filtre 12 présentent, quant à eux, une aire d'ouverture au plus égale, à 7,1 mm².

L'installation 1 comprend encore une pièce 24 amovible configurée pour, à l'état positionné à l'intérieur du corps 6 du réacteur et en position fermée de l'élément 100 mobile de fermeture de la sortie 4 d'évacuation des produits traités, former une pièce entretoise entre la partie du filtre 12 portée par l'élément 100 mobile de fermeture de la sortie 4 d'évacuation des produits traités du réacteur 2, et la ou les cassettes disposées à l'intérieur du réacteur 2.

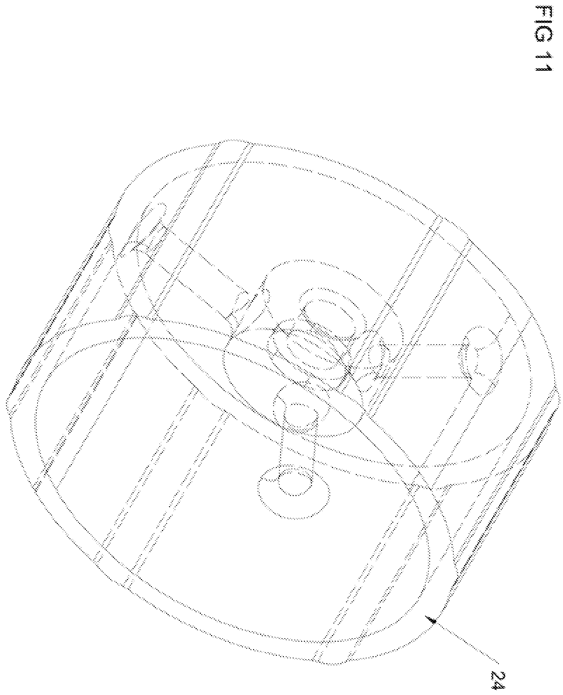

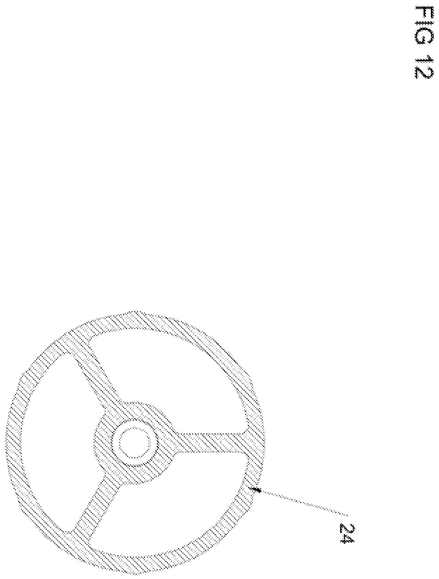

Cette pièce entretoise 24 est représentée en détail notamment aux figures 11 et 12. Cette pièce entretoise 24 se présente sous forme d'un tronçon de cylindre muni intérieurement d'une conduite centrale raccordée à la paroi périphérique extérieure de délimitation du cylindre par des bras radiaux.

La conduite centrale de cette pièce entretoise 24 assure, à l'état fermé du réacteur contenant au moins une cassette, l'interface entre la conduite centrale de la cassette la plus proche de la sortie 4 d'évacuation des produits traités du réacteur et le sommet du cône 22 à surface poreuse du filtre 12.

Dans les exemples représentés, la conduite centrale de chaque cassette est poreuse, ce qui facilite l'amenée de l'inoculum au cœur des produits à traiter.

Cette conduite 20 centrale aurait pu être étanche, c'est-à-dire avec une paroi pleine, sans sortir du cadre de l'invention. Dans ce cas, la partie active de filtration du filtre aurait été limitée à la surface poreuse du cône 22.

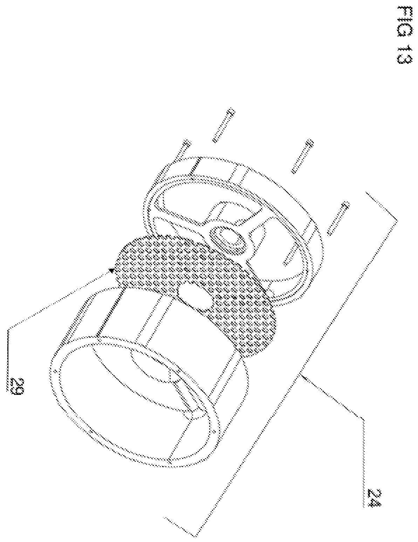

Cette pièce entretoise 24 peut, comme l'illustre la figure 13, intégrer un élément filtrant 29 évidé centralement et se présentant ici sous forme d'une plaque perforée.

En pratique, le fonctionnement d'une telle installation est le suivant.

On suppose que des cassettes ont été remplies de produits à traiter et introduites dans le réacteur par l'entrée 3 d'alimentation en produit à traiter du réacteur pour former une ligne de cassettes à l'intérieur du réacteur, comme illustré à la figure 2.

Généralement, le réacteur est disposé à la verticale avec l'entrée 3 d'alimentation en produits à traiter disposée au-dessus de la sortie de 4 d'évacuation de produits traités.

Un cycle d'immersion, tel que décrit ci-dessus, peut alors être lancé. Ce cycle comprend une étape d'alimentation en inoculum du réacteur par pompage d'inoculum dans le réservoir de stockage. L'inoculum est introduit dans le réacteur à travers l'orifice 8 d'entrée d'inoculum. Une partie de cet inoculum traverse le cône 22 à surface perforée. Une autre partie traverse la conduite centrale des cassettes lorsque cette dernière est perforée pour parvenir dans tous les cas, dans la zone 110 de stockage de produits des cassettes 11.

En effet, le fluide, issu du circuit 16 de circulation pénètre dans le réacteur 2 via l'orifice 8 d'entrée d'inoculum, alimente chaque cassette via sa conduite centrale ou son couvercle et percole à travers les produits solides à traiter de chaque cassette. Il passe ainsi à travers les produits solides contenus dans les cassettes jusqu'à atteindre la cassette la plus éloignée de l'orifice 8 d'entrée d'inoculum.

La distribution de l'inoculum dans les zones 110 de stockage de produits des cassettes s'opère à travers les pores de la cassette. Une fois le niveau souhaité de remplissage du réacteur atteint l'alimentation en inoculum est stoppée.

Au contact des produits solides à traiter, l'inoculum se charge en matière organique solubilisée, c'est-à-dire en matière organique solide dégradée et liquéfiée.

Puis, après une période de temps prédéterminée de contact des produits avec l'inoculum, le réacteur est vidé via l'orifice 9 de sortie de lixiviat après que le lixiviat soit passé à travers le filtre 12, et le lixiviat est collecté dans le réservoir de stockage pour former l'inoculum du cycle d'immersion suivant.

A l'issue d'un cycle d'immersion, il peut être décidé de remplacer une ou plusieurs cassettes contenues dans le réacteur. Il suffit alors :
- d'amener le réacteur en position horizontale via son système d'entraînement en déplacement à pivotement,
- de préparer un bac à positionner sous l'élément de fermeture de la sortie 4 d'évacuation des produits traités afin de récupérer le lixiviat stagnant dans le réacteur tubulaire lors de l'ouverture dudit élément de fermeture,
- de remettre ce lixiviat dans le réservoir de stockage,
- d'ouvrir l'élément de fermeture de l'entrée 3 d'alimentation en produits à traiter,
- d'enlever la pièce entretoise,
- d'introduire une cassette chargée en produits à traiter dans le réacteur par l'entrée 3 d'alimentation en produits à traiter,
- de récupérer au niveau de la sortie 5 d'évacuation des produits traités, la cassette expulsée sous l'effet d'une poussée exercée sur la ligne de cassettes lors de l'introduction de la nouvelle cassette,
- de nettoyer le cône 22 à surface poreuse,
- de replacer la pièce entretoise 24 au niveau de l'élément 100 de fermeture de la sortie 4 d'évacuation de produits traités,
- de refermer ledit élément 100 de fermeture de la sortie 4 d'évacuation des produits traités, et
- de basculer le réacteur en position verticale, le réacteur étant prêt pour un nouveau cycle d'immersion.

Selon les modalités de pilotage choisies, le remplissage et la vidange en liquide du réacteur peuvent être réalisés de façon automatique ou manuelle. Dans le cas d'une gestion automatique, l'unité du pilotage qui se présente sous la forme d'un système électronique et informatique, comprend par exemple un microprocesseur et une mémoire de travail. Selon un aspect particulier, l'unité de pilotage peut se présenter sous la forme d'un automate programmable. Autrement dit, les fonctions et étapes décrites peuvent être mise en œuvre sous forme de programme informatique ou via des composants matériels (p. ex. des réseaux de portes programmables). En particulier, les fonctions et étapes opérées par l'unité de pilotage ou ses modules peuvent être réalisées par des jeux d'instructions ou modules informatiques implémentés dans un processeur ou contrôleur ou être réalisées par des composants électroniques dédiés ou des composants de type circuit logique programmable (ou FPGA qui est l'acronyme de l'anglais field-programmable gate array, ce qui correspond littéralement à réseau de portes programmable in-situ) ou de type circuit intégré propre à une application (ou ASIC qui est l'acronyme de l'anglais application-specific integrated circuit, ce qui correspond littéralement à circuit intégré spécifique à une application). Il est aussi possible de combiner des parties informatiques et des parties électroniques. Lorsqu'il est précisé que l'unité ou des moyens ou modules de ladite unité sont configurés pour réaliser une opération donnée, cela signifie que l'unité comprend des instructions informatiques et les moyens d'exécution correspondants qui permettent de réaliser ladite opération et/ou que l'unité comprend des composants électroniques correspondants.

## Revendications

1. Installation (1) de traitement, notamment pour le traitement par fermentation de produits solides au moins partiellement fermentescibles, en particulier de déchets organiques solides, en vue notamment de la production de biogaz, ladite installation (1) comprenant un réacteur (2) tubulaire, un châssis support (26) dudit réacteur (2) tubulaire, et au moins une cassette (11) présentant une zone (110) ajourée de stockage de produit, ledit réacteur (2) tubulaire étant équipé au moins d'une entrée (3) d'alimentation en produits à traiter, d'une première sortie (4), dite sortie (4) d'évacuation des produits traités, d'une deuxième sortie (5), dite sortie (5) d'évacuation de biogaz, d'un orifice (8) d'entrée de liquide, dit orifice (8) d'entrée d'un inoculum, d'un orifice (9) de sortie de liquide, dit orifice (9) de sortie de lixiviat, ledit réacteur (2) tubulaire comprenant un corps (6) tubulaire et un séparateur (7) liquide/solide, ledit corps (6) tubulaire étant fermé à chacune de ses extrémités par un dispositif (10) de fermeture étanche à l'air, ce dispositif (10) de fermeture comprenant au moins un élément (100) de fermeture monté mobile entre une position ouverte et une position fermée de ladite extrémité, lesdites extrémités du corps (6) formant l'une, l'entrée (3) d'alimentation en produits à traiter, l'autre, la sortie (4) d'évacuation des produits traités dudit réacteur (2), et la ou chaque cassette (11) étant montée déplaçable axialement à coulissement à l'intérieur du corps (6) depuis l'entrée (3) d'alimentation en produits à traiter vers la sortie (4) d'évacuation des produits traités, **caractérisée en ce que** le séparateur (7) liquide/solide comprend un filtre (12) présentant une partie active de filtration (121) configurée pour, en position fermée de l'élément (100) mobile de fermeture de la sortie (4) d'évacuation des produits traités dudit réacteur (2) et à l'état positionné d'au moins une cassette (11) dans le réacteur (2), s'interposer entre les orifices (8) d'entrée d'un inoculum et (9) de sortie de lixiviat et la zone de stockage (110) de la ou de chaque cassette (11) disposée à l'intérieur du réacteur (2), et **en ce que** l'orifice (8) d'entrée d'un inoculum, l'orifice (9) de sortie de lixiviat et au moins une partie du filtre (12) sont portés par l'élément (100) mobile de fermeture de la sortie (4) d'évacuation des produits traités dudit réacteur (2).

2. Installation (1) de traitement, notamment pour le traitement par fermentation de produits solides au moins partiellement fermentescibles, selon la revendication 1, **caractérisée en ce que** l'orifice (8) d'entrée d'un inoculum, l'orifice (9) de sortie de lixiviat sont communs et formés par un même orifice.

3. Installation (1) de traitement, notamment pour le traitement par fermentation de produits solides au moins partiellement fermentescibles selon l'une des revendications 1 ou 2, **caractérisée en ce que** le corps (6) tubulaire du réacteur (2) est un corps cylindrique de révolution et **en ce que**, en position fermée de l'élément (100) mobile de fermeture de la sortie (4) d'évacuation des produits traités du réacteur (2), l'axe longitudinal central du corps (6) tubulaire cylindrique du réacteur (2) passe par l'orifice (8) d'entrée d'un inoculum et l'orifice (9) de sortie de lixiviat.

4. Installation (1) de traitement, notamment pour le traitement par fermentation de produits solides au moins partiellement fermentescibles selon l'une des revendications 1 à 3, **caractérisée en ce que** l'installation (1) comprend un réservoir (15) de stockage de liquide disposé à l'extérieur du réacteur (2) et un circuit (16) de circulation de fluide pour le raccordement dudit réservoir (15) à l'orifice (8) d'entrée d'un inoculum et à l'orifice (9) de sortie de lixiviat.

5. Installation (1) de traitement, notamment pour le traitement par fermentation de produits solides au moins partiellement fermentescibles selon la revendication 4, **caractérisée en ce que** l'installation comprend une pompe (17) à inversion de sens disposée sur ledit circuit (16) de circulation de fluide et une unité (18) de pilotage de ladite pompe (17).

6. Installation (1) de traitement, notamment pour le traitement par fermentation de produits solides au moins partiellement fermentescibles selon l'une des revendications 1 à 5, **caractérisée en ce que**, à l'état fermé du réacteur (2) et inséré de la ou les cassettes (11) dans le réacteur (2), la partie du filtre (12) portée par l'élément (100) mobile de fermeture de la sortie (4) d'évacuation des produits traités dudit réacteur (2) se prolonge par un conduit (19) axial poreux disposé à l'intérieur du réacteur (2).

7. Installation (1) de traitement, notamment pour le traitement par fermentation de produits solides au moins partiellement fermentescibles selon l'une des revendications 1 à 6, **caractérisée en ce que** la ou au moins l'une des cassettes (11) est une cassette cylindrique munie intérieurement d'une conduite (20) centrale s'étendant au moins depuis une extrémité du cylindre jusqu'à l'extrémité opposée.

8. Installation (1) de traitement, notamment pour le traitement par fermentation de produits solides au moins partiellement fermentescibles selon la revendication 7, prise en combinaison avec la revendication 6, **caractérisée en ce que** la conduite (20) centrale de la cassette (11) est une conduite poreuse, ladite conduite (20) formant au moins une partie du conduit (19) axial poreux disposé à l'intérieur du réacteur (2).

9. Installation (1) de traitement, notamment pour le traitement par fermentation de produits solides au moins partiellement fermentescibles selon l'une des revendications 7 ou 8, **caractérisée en ce que** ladite cassette (11) cylindrique comprend un corps (111) cylindrique délimité par une paroi (1110) ajourée et au moins deux couvercles (112) ajourés de fermeture l'un, d'une extrémité, l'autre de l'extrémité opposée du cylindre formé par le corps (111), **en ce que** la paroi (1110) de délimitation du corps (111) est écartée de la conduite (20) centrale pour définir un espace formant la zone (110) ajourée de stockage de produit de la cassette (11), et **en ce que** les extrémités de la conduite (20) centrale sont de formes complémentaires.

10. Installation (1) de traitement, notamment pour le traitement par fermentation de produits solides au moins partiellement fermentescibles selon la revendication 9, **caractérisée en ce que** le filtre (12) présente des pores (21) dont l'aire d'ouverture est prédéterminée et **en ce que** chaque ajour (27) de la paroi (1110) de délimitation du corps (111) de la cassette (11) et chaque ajour (28) des couvercles (112) de fermeture présentent une aire d'ouverture supérieure à l'aire d'ouverture de l'un quelconque des pores (21) du filtre (12).

11. Installation (1) de traitement, notamment pour le traitement par fermentation de produits solides au moins partiellement fermentescibles selon l'une des revendications 1 à 10, **caractérisée en ce que** la partie du filtre (12) portée par l'élément (100) mobile de fermeture de la sortie (4) d'évacuation des produits traités dudit réacteur (2) est formée de deux cônes (22, 23) à conicité inversée pour se raccorder par la base (220, 230) des cônes (22, 23), les sommets (221, 231) des cônes(22, 23) étant disposés sur une ligne formant l'axe longitudinal central du corps (6) tubulaire du réacteur (2) en position fermée de l'élément (100) mobile de fermeture de la sortie (4) d'évacuation des produits traités.

12. Installation (1) de traitement, notamment pour le traitement par fermentation de produits solides au moins partiellement fermentescibles selon la revendication 11, **caractérisée en ce que** l'un (22) des cônes (22, 23) formant au moins une partie de la partie active de filtration (121) du filtre (12) est délimité par une surface poreuse, **en ce que** l'autre cône (23) est délimité par une surface pleine et **en ce que** les orifices (8) d'entrée d'un inoculum et (9) de sortie de lixiviat sont disposés au niveau du sommet (231) du cône (23) à surface pleine.

13. Installation (1) de traitement, notamment pour le traitement par fermentation de produits solides au moins partiellement fermentescibles selon l'une des revendications 1 à 12, **caractérisée en ce que** l'installation comprend au moins une pièce (24) amovible configurée pour, à l'état positionné à l'intérieur du corps (6) du réacteur (2) en position fermée de l'élément (100) mobile de fermeture de la sortie (4) d'évacuation des produits traités, former une pièce entretoise entre la partie du filtre (12) portée par l'élément (100) mobile de fermeture de la sortie (4) d'évacuation des produits traités dudit réacteur (2) et la ou les cassettes (11) disposées à l'intérieur du réacteur (2).

## Patentansprüche

1. Behandlungsanlage (1), insbesondere zur fermentierenden Behandlung zumindest teilweise fermentierbarer Feststoffen, insbesondere von festen organischen Abfällen, um Biogas zu erzeugen, wobei die Anlage (1) einen Rohrreaktor (2), ein Stützgestell (26) für den Rohrreaktor (2) und mindestens eine Kassette (11) umfasst, die einen durchbrochenen Stofflagerungsbereich (110) aufweist, wobei der Rohrreaktor (2) mit mindestens einer Eintrittsöffnung (3) zum Einspeisen zu behandelnder Stoffe, mit einer ersten Austrittsöffnung (4), die als Austrittsöffnung (4) zur Entnahme der behandelten Stoffe bezeichnet wird, mit einer zweiten Austrittsöffnung (5), die Austrittsöffnung (5) zur Entnahme von Biogas bezeichnet wird, mit einer Öffnung (8) zum Einleiten von Flüssigkeit, die als Öffnung (8) zum Einleiten eines Beimpfungsansatzes bezeichnet wird, mit einer Öffnung (9) zum Abführen von Flüssigkeit ausgestattet ist, die als Öffnung (9) zum Abführen von Sickerflüssigkeit bezeichnet wird, wobei der Rohrreaktor (2) einen Rohrkörper (6) und einen FLüssigkeits/Feststoff-Abscheider (7) umfasst, wobei der Rohrkörper (6) an jedem seiner Enden mit einer luftdichten Schließvorrichtung (10) verschlossen ist, wobei die Schließvorrichtung (10) mindestens ein Schließelement (100) umfasst, das derart angebracht ist, dass es zwischen einer offenen Stellung und einer geschlossenen Stellung des Endes beweglich ist, wobei eines von den Enden des Körpers (6) die Eintrittsöffnung (3) zum Einspeisen zu behandelnder Stoffe und das andere die Austrittsöffnung (4) zur Entnahme der behandelten Stoffe aus dem Reaktor (2) bildet, und wobei die oder jede der Kassetten (11) derart angebracht ist, dass sie axial gleitend im Inneren des Körpers (6) von der Eintrittsöffnung (3) zum Einspeisen zu behandelnder Stoffe in Richtung der Austrittsöffnung (4) zur Entnahme der behandelten Stoffe verschoben werden kann, **dadurch gekennzeichnet, dass** der Flüssigkeits-/FeststoffAbscheider (7) ein Filter (12) umfasst, welches einen aktiven Filtrationsabschnitt (121) aufweist, der dafür ausgelegt ist, dass er sich, in der geschlossenen Stellung des beweglichen Schließelements (100) der Austrittsöffnung (4) zur Entnahme der behandelten Stoffe aus dem Reaktor (2) und wenn sich mindestens eine Kassette (11) in dem Reaktor (2) befindet, zwischen den Öffnungen (8) zum Einleiten eines Beimpfungsansatzes und (9) zum Abführen von Sickerflüssigkeit und dem Lagerungsbereich (110) der oder jeder der Kassetten (11) befindet, die im Inneren des Reaktors (2) angeordnet ist, und dadurch, dass die Öffnung (8) zum Einleiten eines Beimpfungsansatzes, die Öffnung (9) zum Abführen von Sickerflüssigkeit und zumindest ein Abschnitt des Filters (12) vom beweglichen Schließelement (100) der Austrittsöffnung (4) zur Entnahme der behandelten Stoffe aus dem Reaktor (2) getragen werden.

2. Behandlungsanlage (1), insbesondere zur fermentierenden Behandlung von zumindest teilweise fermentierbaren Feststoffen, gemäß Anspruch 1, **dadurch gekennzeichnet, dass** die Öffnung (8) zum Einleiten eines Beimpfungsansatzes, die Öffnung (9) zum Abführen von Sickerflüssigkeit gemeinsam vorliegen und von ein und derselben Öffnung gebildet werden.

3. Behandlungsanlage (1), insbesondere zur fermentierenden Behandlung von zumindest teilweise fermentierbaren Feststoffen, gemäß einem der Ansprüche 1 oder 2, **dadurch gekennzeichnet, dass** der Rohrkörper (6) des Reaktors (2) einem drehsymmetrischen zylindrischen Körper entspricht, und dadurch, dass die mittige Längsachse des zylindrischen Rohrkörpers (6) des Reaktors (2), in der geschlossenen Stellung des beweglichen Schließelements (100) der Austrittsöffnung (4) zur Entnahme der behandelten Stoffe aus dem Reaktor (2), durch die Öffnung (8) zum Einleiten eines Beimpfungsansatzes und die Öffnung (9) zum Abführen von Sickerflüssigkeit verläuft.

4. Behandlungsanlage (1), insbesondere zur fermentierenden Behandlung von zumindest teilweise fermentierbaren Feststoffen, gemäß einem der Ansprüche 1 bis 3, **dadurch gekennzeichnet, dass** die Anlage (1) einen Vorratsbehälter (15) zur Lagerung von Flüssigkeit, der außerhalb des Reaktors (2) angeordnet ist, und einen Kreislauf (16) zum Umwälzen von Fluid umfasst, um den Vorratsbehälter (15) an die öffnung (8) zum Einleiten eines Beimpfungsansatzes und die Öffnung (9) zum Abführen von Sickerflüssigkeit anzuschließen.

5. Behandlungsanlage (1), insbesondere zur fermentierenden Behandlung von zumindest teilweise fermentierbaren Feststoffen, gemäß Anspruch 4, **dadurch gekennzeichnet, dass** die Anlage eine Pumpe (17) mit Strömungsumkehr, die an dem Kreislauf (16) zum Umwälzen von Fluid angeordnet ist, und eine Einheit (18) zur Steuerung der Pumpe (17) umfasst.

6. Behandlungsanlage (1), insbesondere zur fermentierenden Behandlung von zumindest teilweise fermentierbaren Feststoffen, gemäß einem der Ansprüche 1 bis 5, **dadurch gekennzeichnet, dass** der Abschnitt des Filters (12), der von dem beweglichen Schließelement (100) der Austrittsöffnung (4) zur Entnahme der behandelten Stoffe aus dem Reaktor (2) getragen wird, sich in einer porösen axialen Leitung (19) fortsetzt, die im Inneren des Reaktors (2) angeordnet ist, wenn der Reaktor (2) geschlossen ist und die oder jede der Kassetten (11) in den Reaktor (2) eingeführt ist.

7. Behandlungsanlage (1), insbesondere zur fermentierenden Behandlung von zumindest teilweise fermentierbaren Feststoffen, gemäß einem der Ansprüche 1 bis 6, **dadurch gekennzeichnet, dass** die oder mindestens eine der Kassetten (11) eine zylindrische Kassette ist, die innen mit einer mittigen Leitung (20) versehen ist, welche sich zumindest von einem Ende des Zylinders zum entgegengesetzten Ende erstreckt.

8. Behandlungsanlage (1), insbesondere zur fermentierenden Behandlung von zumindest teilweise fermentierbaren Feststoffen, gemäß Anspruch 7 in Kombination mit dem Anspruch 6, **dadurch gekennzeichnet, dass** die mittige Leitung (20) der Kassette (11) eine poröse Leitung ist, wobei die Leitung (20) zumindest einen Abschnitt der porösen axialen Leitung (19) bildet, welche im Inneren des Reaktors (2) angeordnet ist.

9. Behandlungsanlage (1), insbesondere zur fermentierenden Behandlung von zumindest teilweise fermentierbaren Feststoffen, gemäß einem der Ansprüche 7 oder 8, **dadurch gekennzeichnet, dass** die zylindrische Kassette (11) einen zylindrischen Körper (111), der von einer durchbrochenen Wand (1110) begrenzt wird, und mindestens zwei durchbrochene Deckel (112) umfasst, von denen der eine zum Schließen eines Endes und der andere zum Schließen des entgegengesetzten Endes des Zylinders dient, welcher von dem Körper (111) gebildet wird, dadurch, dass die Wand (1110) zur Begrenzung des Körpers (111) von der mittigen Leitung (20) beabstandet ist, um eine Raum festzulegen, welcher den durchbrochenen Stofflagerungsbereich (110) der Kassette (11) bildet, und dadurch, dass die Enden der mittigen Leitung (20) sich ergänzende Formen haben.

10. Behandlungsanlage (1), insbesondere zur fermentierenden Behandlung von zumindest teilweise fermentierbaren Feststoffen, gemäß Anspruch 9, **dadurch gekennzeichnet, dass** das Filter (12) Poren (21) aufweist, deren Öffnungsfläche vorbestimmt ist, und dadurch, dass jeder Durchbruch (27) der Wand (1110) zur Begrenzung des Körpers (111) der Kassette (11) und jeder Durchbruch (28) der Schließdeckel (112) eine Öffnungsfläche aufweisen, die größer als die Öffnungsfläche einer beliebigen der Poren (21) des Filters (12) ist.

11. Behandlungsanlage (1), insbesondere zur fermentierenden Behandlung von zumindest teilweise fermentierbaren Feststoffen, gemäß einem beliebigen der Ansprüche 1 bis 10, **dadurch gekennzeichnet, dass** der Abschnitt des Filters (12), welcher von dem beweglichen Schließelement (100) der Austrittsöffnung (4) zur Entnahme der behandelten Stoffe aus dem Reaktor (2) getragen wird, von zwei Kegeln (22, 23) gebildet wird, die eine umgekehrte Kegelform aufweisen, um sich über die Basis (220, 230) der Kegel (22, 23) aneinanderzufügen, wobei die Spitzen (221, 231) der Kegel (22, 23) auf einer Linie angeordnet sind, welche die mittige Längsachse des Rohrkörpers (6) des Reaktors (2) bildet, wenn das bewegliche Schließelement (100) der Austrittsöffnung (4) zur Entnahme der behandelten Stoffe seine geschlossene Stellung einnimmt.

12. Behandlungsanlage (1), insbesondere zur fermentierenden Behandlung von zumindest teilweise fermentierbaren Feststoffen, gemäß Anspruch 11, **dadurch gekennzeichnet, dass** einer (22) der Kegel (22, 23), welcher zumindest einen Abschnitt des aktiven Filtrationsabschnitts (121) des Filters (12) bildet, von einer porösen Fläche begrenzt wird, dadurch, dass der andere Kegel (23) von einer durchgehenden Fläche begrenzt wird, und dadurch, dass die Öffnungen (8) zum Einleiten eines Beimpfungsansatzes und (9) zum Abführen von Sickerflüssigkeit auf Höhe der Spitze (231) des Kegels (23) mit durchgehender Fläche angeordnet sind.

13. Behandlungsanlage (1), insbesondere zur fermentierenden Behandlung von zumindest teilweise fermentierbaren Feststoffen, gemäß einem beliebigen der Ansprüche 1 bis 12, **dadurch gekennzeichnet, dass** die Anlage mindestens ein bewegliches Teil (24) umfasst, das dafür ausgelegt ist, wenn es im Inneren des Körpers (6) des Reaktors (2) angeordnet ist und das bewegliche Schließelement (100) der Austrittsöffnung (4) zur Entnahme der behandelten Stoffe seine geschlossene Stellung einnimmt, eine Querverstrebung zwischen dem Abschnitt des Filters (12), der von dem Schließelement (100) der Austrittsöffnung (4) zur Entnahme der behandelten Stoffe aus dem Reaktor (2) getragen wird, und der oder jeder der Kassetten (11) zu bilden, welche im Inneren des Reaktors (2) angeordnet sind.

## Claims

1. A treatment facility (1), in particular for the treatment of at least partially fermentable solid products, particularly solid organic waste, by fermentation, in particular with a view to producing biogas, said facility (1) comprising a tubular reactor (2), a frame (26) for supporting said tubular reactor (2), and at least one cartridge (11) having a perforated product storage area (110), said tubular reactor (2) being provided at least with a feed inlet (3) for products to be treated, a first outlet (4), referred to as the discharge outlet (4) for treated products, a second outlet (5), referred to as the biogas discharge outlet (5), a liquid inlet orifice (8), referred to as the inoculum inlet orifice (8), and a liquid outlet orifice (9), referred to as the leachate outlet orifice (9), said tubular reactor (2) comprising a tubular body (6) and a liquid-solid separator (7), said tubular body (6) being closed at each of its ends by an airtight closure device (10), this closure device (10) comprising at least one closure element (100) movably mounted between an open position and a closed position of said end, one of said ends of the body (6) forming the feed inlet (3) for products to be treated and the other forming the discharge outlet (4) for treated products of said reactor (2), and the or each cartridge (11) being axially slidably mounted inside the body (6) from the feed inlet (3) for products to be treated toward the discharge outlet (4) for treated products, **characterized in that** the liquid-solid separator (7) comprises a filter (12) having an active filtration portion (121) configured, in the closed position of the movable closure element (100) of the discharge outlet (4) for treated products of said reactor (2) and in the state in which at least one cartridge (11) is positioned in the reactor (2), to be interposed between the inoculum inlet orifice (8) and the leachate outlet orifice (9), and the storage area (110) of the or each cartridge (11) arranged inside the reactor (2), and **in that** the inoculum inlet orifice (8), the leachate outlet orifice (9), and at least one portion of the filter (12), are borne by the movable closure element (100) of the discharge outlet (4) for treated products of said reactor (2).

2. The treatment facility (1), in particular for the treatment of at least partially fermentable solid products by fermentation, as claimed in claim 1, **characterized in that** the inoculum inlet orifice (8) and the leachate outlet orifice (9) are common and formed by a single orifice.

3. The treatment facility (1), in particular for the treatment of at least partially fermentable solid products by fermentation, as claimed in one of claims 1 and 2, **characterized in that** the tubular body (6) of the reactor (2) is a cylindrical body of revolution and **in that**, in the closed position of the movable closure element (100) of the discharge outlet (4) for treated products of the reactor (2), the central longitudinal axis of the cylindrical tubular body (6) of the reactor (2) passes through the inoculum inlet orifice (8) and the leachate outlet orifice (9).

4. The treatment facility (1), in particular for the treatment of partially fermentable solid products by fermentation, as claimed in one of claims 1 to 3, **characterized in that** the facility (1) comprises a liquid storage tank (15) arranged outside the reactor (2) and a fluid circulation circuit (16) for connecting said tank (15) to the inoculum inlet orifice (8) and the leachate outlet orifice (9).

5. The treatment facility (1), in particular for the treatment of at least partially fermentable solid products by fermentation, as claimed in claim 4, **characterized in that** the facility comprises a reversible pump (17) arranged on said fluid circulation circuit (16), and a unit (18) for controlling said pump (17).

6. The treatment facility (1), in particular for the treatment of at least partially fermentable solid products by fermentation, as claimed in one of claims 1 to 5, **characterized in that**, in the state in which the reactor (2) is closed and the cartridge or cartridges (11) is/are inserted into the reactor (2), the portion of the filter (12) borne by the movable closure element (100) of the discharge outlet (4) for treated products of the reactor (2) is extended by a porous axial duct (19) arranged inside the reactor (2).

7. The treatment facility (1), in particular for the treatment of at least partially fermentable solid products by fermentation, as claimed in one of claims 1 to 6, **characterized in that** the cartridge or at least one of the cartridges (11) is a cylindrical cartridge internally provided with a central duct (20) extending at least from one end of the cylinder to the opposite end.

8. The treatment facility (1), in particular for the treatment of at least partially fermentable solid products by fermentation, as claimed in claim 7 taken in combination with claim 6, **characterized in that** the central duct (20) of the cartridge (11) is a porous duct, said duct (20) forming at least part of the porous axial duct (19) arranged inside the reactor (2).

9. The treatment facility (1), in particular for the treatment of at least partially fermentable solid products by fermentation, as claimed in one of claims 7 and 8, **characterized in that** said cylindrical cartridge (11) comprises a cylindrical body (111) delimited by a perforated wall (1110) and at least two perforated covers (112), one for closing one end and the other for closing the opposite end of the cylinder formed by the body (111), **in that** the wall (1110) defining the body (111) is spaced apart from the central duct (20) in order to define a space forming the perforated product storage area (110) of the cartridge (11), and **in that** the ends of the central duct (20) have complementary shapes.

10. The treatment facility (1), in particular for the treatment of at least partially fermentable solid products by fermentation, as claimed in claim 9, **characterized in that** the filter (12) has pores (21) the open area of which is predetermined and **in that** each opening (27) of the wall (1110) defining the body (111) of the cartridge (11) and each opening (28) of the closure covers (112) has an open area greater than the open area of any one of the pores (21) of the filter (12).

11. The treatment facility (1), in particular for the treatment of at least partially fermentable solid products by fermentation, as claimed in one of claims 1 to 10, **characterized in that** the portion of the filter (12) borne by the movable closure element (100) of the discharge outlet (4) for treated products of said reactor (2) is formed by two cones (22, 23) the taper of which is inverted relative to each other so that they are connected by the base (220, 230) of the cones (22, 23), the vertices (221, 231) of the cones (22, 23) being arranged on a line forming the central longitudinal axis of the tubular body (6) of the reactor (2) in the closed position of the movable closure element (100) of the discharge outlet (4) for treated products.

12. The treatment facility (1), in particular for the treatment of at least partially fermentable solid products by fermentation, as claimed in claim 11, **characterized in that** one (22) of the cones (22, 23) forming at least part of the active filtration portion (121) of the filter (12) is delimited by a porous surface, **in that** the other cone (23) is delimited by a solid surface, and **in that** the inoculum inlet orifice (8) and leachate outlet orifice (9) are arranged at the vertex (231) of the cone (23) having a solid surface.

13. The treatment facility (1), in particular for the treatment of at least partially fermentable solid products by fermentation, as claimed in one of claims 1 to 12, **characterized in that** the facility comprises a removable part (24) configured, in the state in which it is positioned inside the body (6) of the reactor (2) and in the closed position of the movable closure element (100) of the discharge outlet (4) for treated products, to form a spacer between the filter portion (12) borne by the movable closure element (100) of the discharge outlet (4) for treated products of said reactor (2), and the cartridge or cartridges (11) arranged inside the reactor (2).
